# EUROPEAN PATENT APPLICATION

(11) **EP 4 495 132 A1**
(43) Date of publication of application: **22.01.2025**
(21) Application number: 24189926.9
(22) Date of filing: 21.07.2024
(51) Int. Cl.: C07K 14/47, C12N 9/22, C12N 15/70

(54) **TARGET GENE EXPRESSION CONTROL SYSTEM COMPRISING DXCAS9 AND CRP DERIVATIVE AND PREPARATION METHOD THEREFOR**

(30) Priority: 21.07.2023 KR 20230095429
(71) Applicant: Korea Research Institute of Chemical Technology, Daejeon 34114 (KR)
(72) Inventor: LEE, Ju Young, 34114 Daejeon (KR); KIM, Mi Ri, 34114 Daejeon (KR); NOH, Myung Hyun, 34114 Daejeon (KR); MOON, Soo Young, 34114 Daejeon (KR)
(74) Representative: Gassner, Birgitta

(57) **Abstract**

Disclosed are a plasmid comprising dxCas9 and a CRP derivative for controlling the expression of a target gene, a recombinant strain transformed with the plasmid, a preparation method therefor, a target gene control system, and a method for controlling the expression of a target gene, wherein the plasmid, recombinant strain, and expression control system according to the present invention can improve the production of high-value-added substances by construction of a system for simultaneously and multiply controlling the expression of target genes.

## Description

### Technical Field

The present invention relates to a plasmid comprising dxCas9 and a CRP derivative for controlling the expression of a target gene, a recombinant strain transformed with the plasmid, a preparation method therefor, a target gene control system, and a method for controlling the expression of a target gene.

### Background Art

In recent years, technology that enables the efficient production of products that wild-type microorganisms cannot produce, by metabolic engineering of industrial microorganisms has been widely used in order to produce high-value-added products in bio-industries. The key of this technology is that a target material needs to be produced efficiently and economically. Examples of specifically used metabolic engineering methods comprise inserting heterologous genes into plasmids or genomes or creating mutations in specific genes. In recent years, technologies that can quickly achieve the insertion or mutations of target genes by using CRISPR have received great interest.

Since CRISPR technology has the possibility to target genes inherent in E. *coli* or foreign genes simultaneously and multiply, a CRISPR-based gene expression control system has great potential as a synthetic biology tool that can improve the production of high-value materials through E. *coli.* However, although CRISPR-based gene expression control tools have often been developed in eukaryotes until now, control systems that simultaneously perform gene expression activation/repression in *E. coli* have been relatively rarely reported.

Under this situation, the present inventors developed a novel CRISPR-based gene expression control system that can simultaneously target multiple genes in E. *coli* and perform transcriptional activation/inhibition functions, and thus completed the invention.

### Disclosure

### Technical Problem

An aspect of the present invention is to provide a plasmid for controlling the expression of a target gene, the plasmid comprising dxCas9 and a CRP derivative.

Another aspect of the present invention is to provide a recombinant strain transformed with the plasmid.

Still another aspect of the present invention is to provide a system for controlling the expression of a target gene, the system comprising dxCas9 and a CRP derivative.

Still another aspect of the present invention is to provide a method for preparing a recombinant strain controlling the expression of a target gene, the method comprising: i) constructing a dxCas9-CRP system in which a dxCas9 protein is bound to a CRP protein; ii) cloning a fluorescent reporter plasmid in the constructed dxCas9-CRP system; iii) additionally cloning a guide RNA in the dxCas9-CRP system constructed in step ii); and iv) transforming the dxCas9-CRP-gRNA into a strain.

Still another aspect of the present invention is to provide a method for controlling the expression of a target gene, the method comprising applying a CRP derivative to dxCas9.

Still another aspect of the present invention is to provide use of a plasmid, comprising dxCas9 and a CRP derivative, for controlling the expression of a target gene.

Still another aspect of the present invention is to provide use of a system, comprising dxCas9 and a CRP derivative, for controlling the expression of a target gene.

### Technical Solution

The present invention will be specifically described as follows. Each description and exemplary embodiment disclosed herein may also be applied to other descriptions and exemplary embodiments. That is, all combinations of various elements disclosed herein fall within the scope of the present invention. Furthermore, the scope of the present invention is not limited by the specific description below.

Furthermore, a person skilled in the art will recognize or be able to ascertain, by using no more than routine experimentation, many equivalents to the specific embodiments of the present invention described herein. Furthermore, these equivalents are intended to be included in the present invention.

In accordance with an aspect of the present invention, there is provided a plasmid for controlling the expression of a target gene, the plasmid comprising dxCas9 and a CRP derivative.

The term "dCas9 (dead Cas9)" as used herein refers to a protein that is capable of sequence-specific recognition/binding but can control gene expression without cleaving target DNA due to the inactivation of nucleolytic activity. The dCas9 can control target gene expression only when it recognizes/binds to the PAM sequence consisting of an NGG sequence in the target gene sequence.

Since dCas9 has a limitation that an NGG sequence needs to exist at the end of a target position in order to select the target site on the target gene sequence, the term "dxCas9" as used herein refers to an evolved variant Cas9 that exhibits high efficiency of specific recognition in a wide range of PAM sequences (NG, NNG, GAA, GAT, CAA) through mutations, meaning a protein having improved PAM compatibility.

In the present invention, the dxCas9 may comprise the amino acid sequence represented by SEQ ID NO: 1, but is not limited thereto.

In the present invention, the dxCas9 may be bound to a linker comprising the amino acid sequence of SEQ ID NO: 2, but is not limited thereto.

In the present invention, the dxCas9 may be linked to CRP or a CRP derivative via a linker. A nucleotide sequence encoding the linker needs to have a sufficient length. Specifically, a nucleotide sequence encoding the linker of the present invention may have a length of at least 20 bp, for example, at least 30 or 40 bp. The nucleotide sequence encoding the linker may have at least about 80% homology, or may have 85%, 86%, 87%, 88%, 89%, 90%, 91%, 92%, 93%, 94%, 95%, 96%, 97%, 98%, or 99% homology with a linear vector.

In the present invention, the linker may comprise the amino acid sequence of GGGAGGGGAG (SEQ ID NO: 2), which is composed of glycine and alanine amino acids, with a small and simple structure, in order to offer structural flexibility to the binding protein, but is not limited thereto.

The term "CRP (cAMP receptor protein)" as used herein refers to a cAMP protein, which is a transcriptional regulatory protein that activates or represses the transcription of 100 or more genes in bacteria. The binding of CRP to DNA activates the transcriptional activity of the target gene by recruiting RNA polymerase. CRP is composed of AR1, AR2, and AR3 (activating regions), which structurally bind to the α-subunit of RNA polymerase to activate gene transcription. CRP is also called a catabolite gene activator protein (CAP). In the present invention, CRP may be used as an effector domain of dxCas9, but is not limited thereto.

The term "derivative" as used herein refers to an analogous protein that is derived by a new combination through deletion, addition, and/or substitution of a specific domain in a wild-type protein. In particular, the derivative may have improved *in vivo* stability, superior storage properties, enhanced activity, or the addition of a separate function, such as comprising binding sites for other components, through deletion, addition, and/or substitution of the amino acids or chemical residues, thereby possessing enhanced or newly added beneficial characteristics as a target protein. The term "CRP derivative" as used herein refers to a derivative in which a specific amino acid sequence has been removed or deleted in the wild-type CRP. Specifically, the CRP protein may lack a DNA-binding motif at the C-terminus or comprise AR1, AR2, and/or AR3, but is not limited thereto.

The CRP derivative of the present invention may be CRP_{AR1}, CRP_{AR3}, CRP_{AR23}, or CRP_{AR123}, and more specifically CRP_{AR123}, but is not limited thereto. CRP_{AR123} may lack a DNA-binding motif at the C-terminus in the wild-type CRP_{WT} consisting of SEQ ID NO: 5, comprise AR1, AR2, and AR3 domains, and consist of the 1st to 180th amino acids, and specifically may comprise SEQ ID NO: 6, but is not limited thereto.

The term "target gene" as used herein refers to a target gene, whose expression level is to be controlled by introduction of a protein fusion. In the present invention, the target gene may mean a single gene or multiple genes and, specifically, may be a gene composed of dxCas9-CRP or -CRP derivative, but is not limited thereto. The "target gene" of the present invention may be used interchangeably with "target gene".

The term "controlling the expression of target gene" as used herein may indicate enhancing or increasing the expression level of the target gene or inhibiting or reducing the expression level thereof, and may also mean a regulatory system capable of dual functions of simultaneously enhancing and inhibiting the expression of the target gene. In the present invention, controlling the expression may be used interchangeably with regulating the expression.

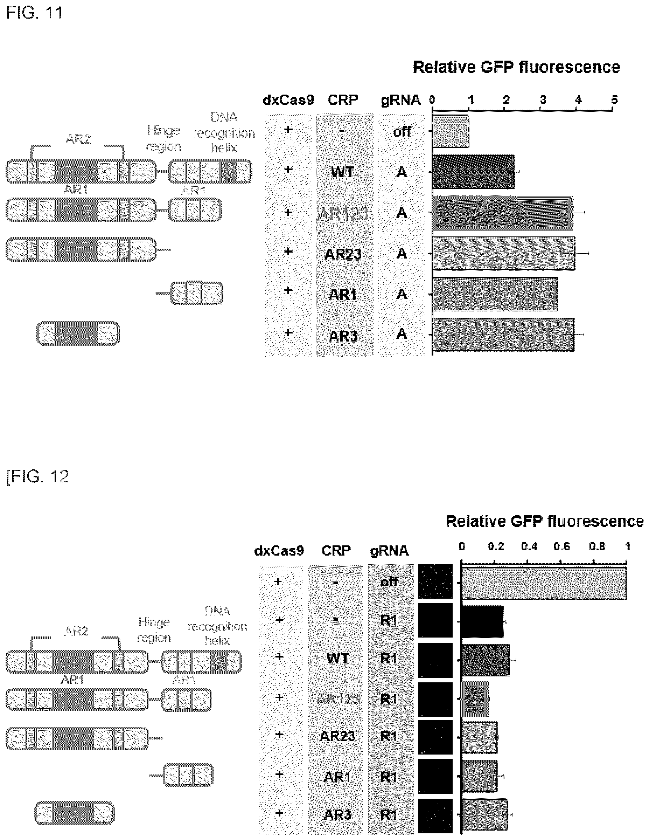

In an embodiment of the present invention, as a result of verifying the effect of enhancing single target gene expression, a dxCas9-CRP_{AR123} derivative expressing strain showed an approximately 4-fold or higher increase in the expression level of GFP fluorescence compared with control strains (FIG. 11), and in an experiment on expression inhibiting effects, showed an approximately 84% or more reduction in the expression level of GFP fluorescence compared with control strains (FIG. 12).

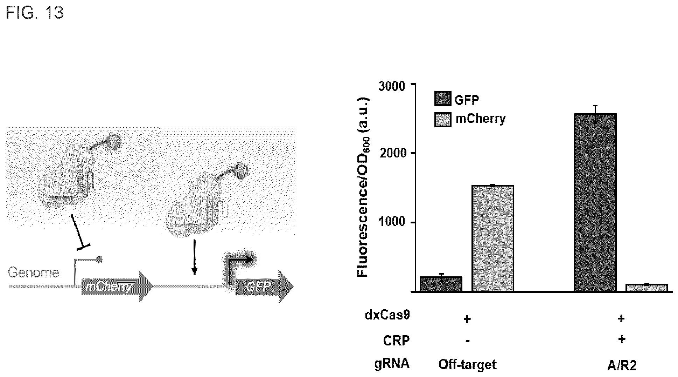

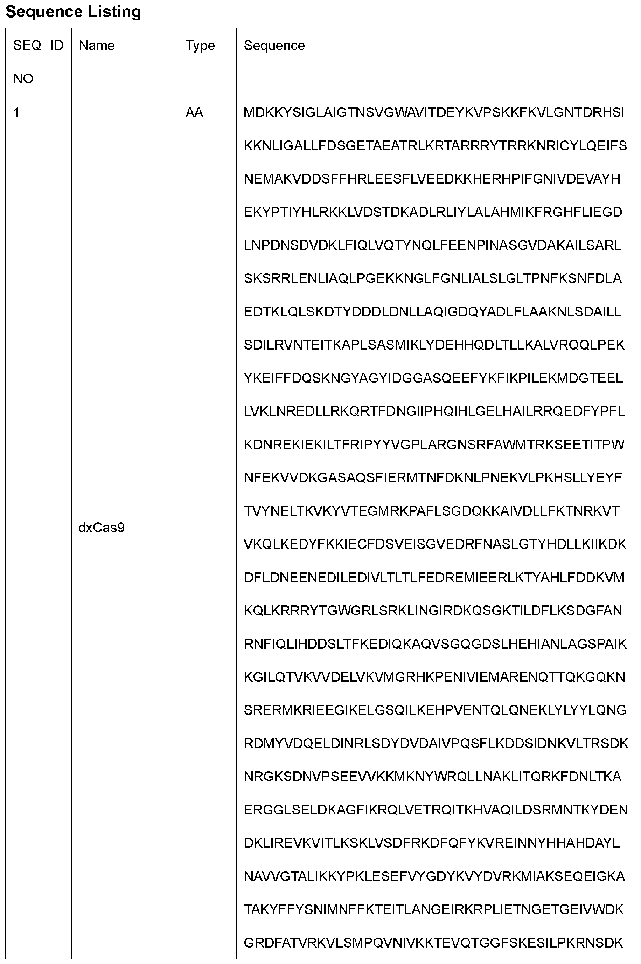

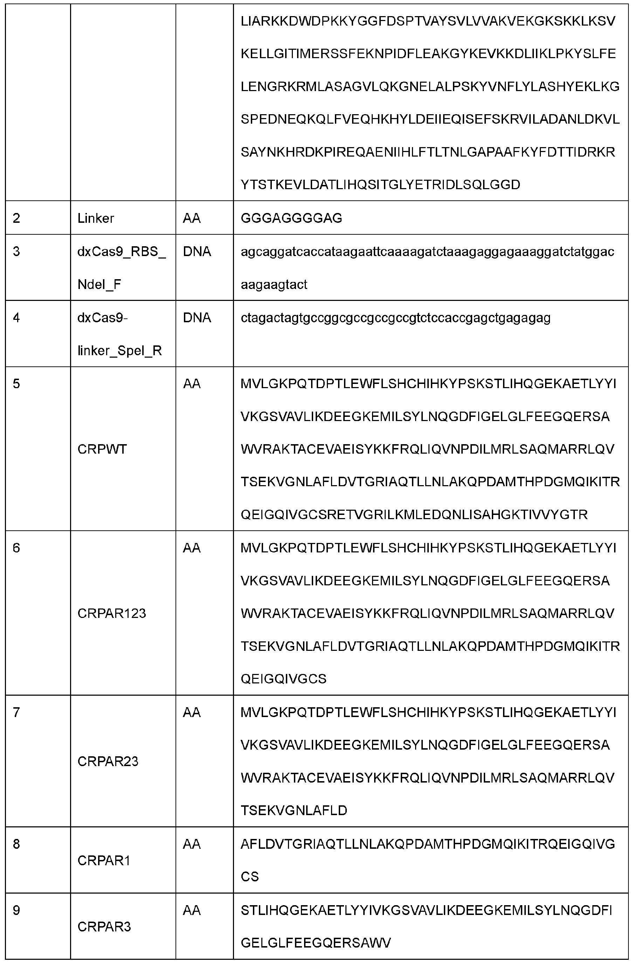

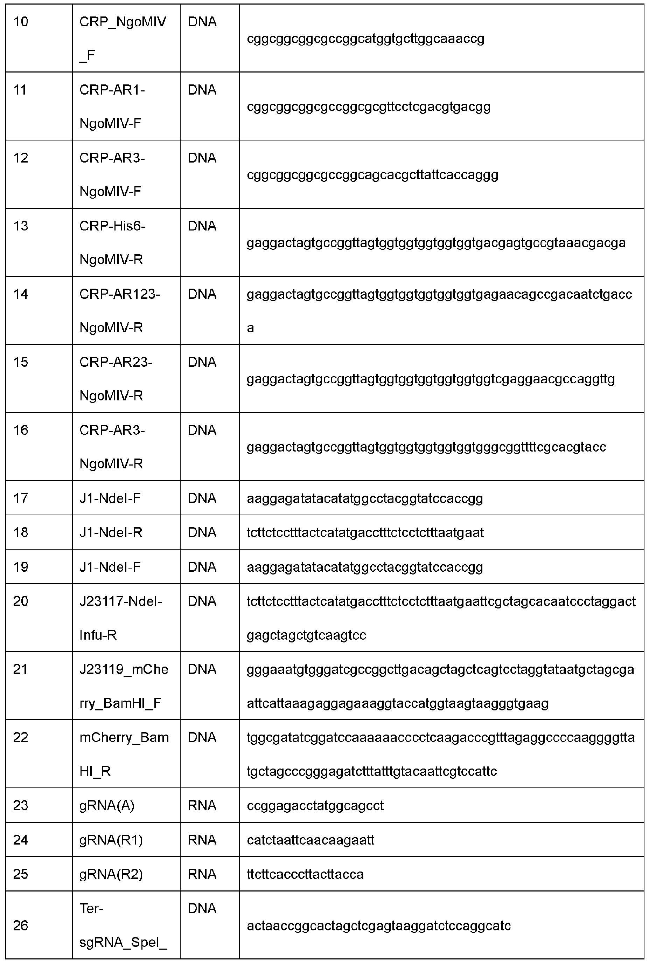

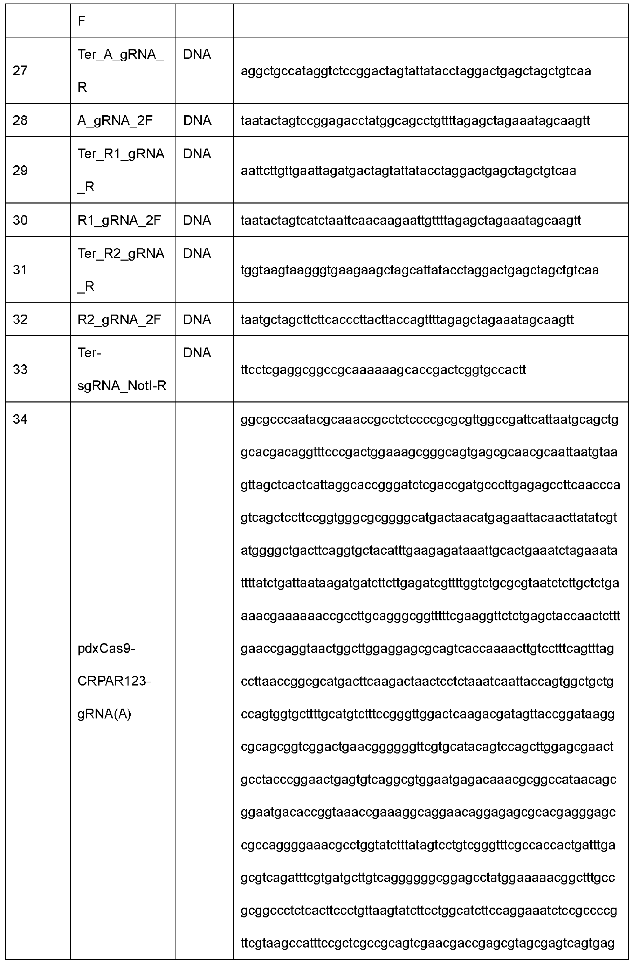

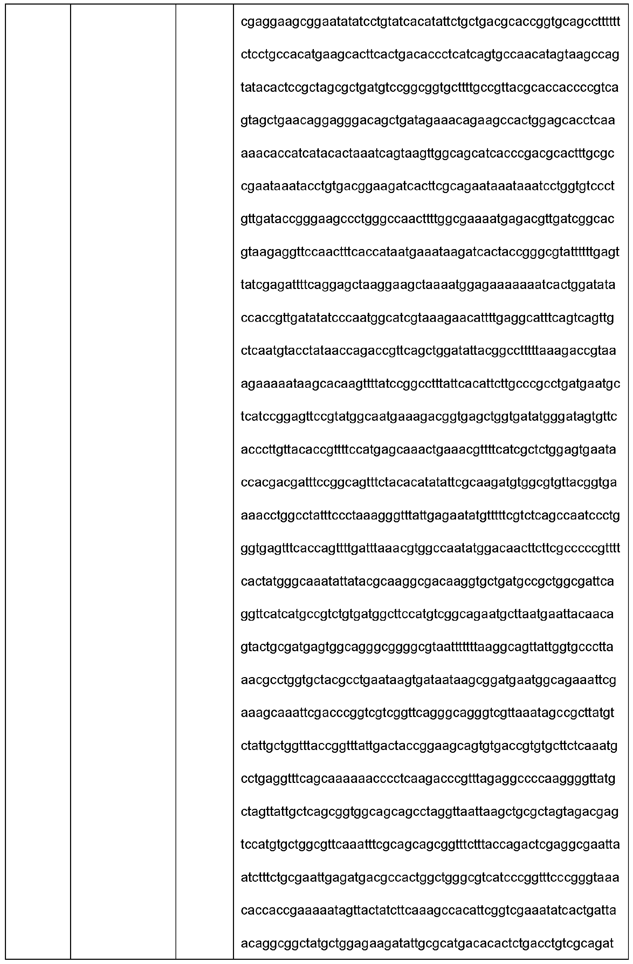

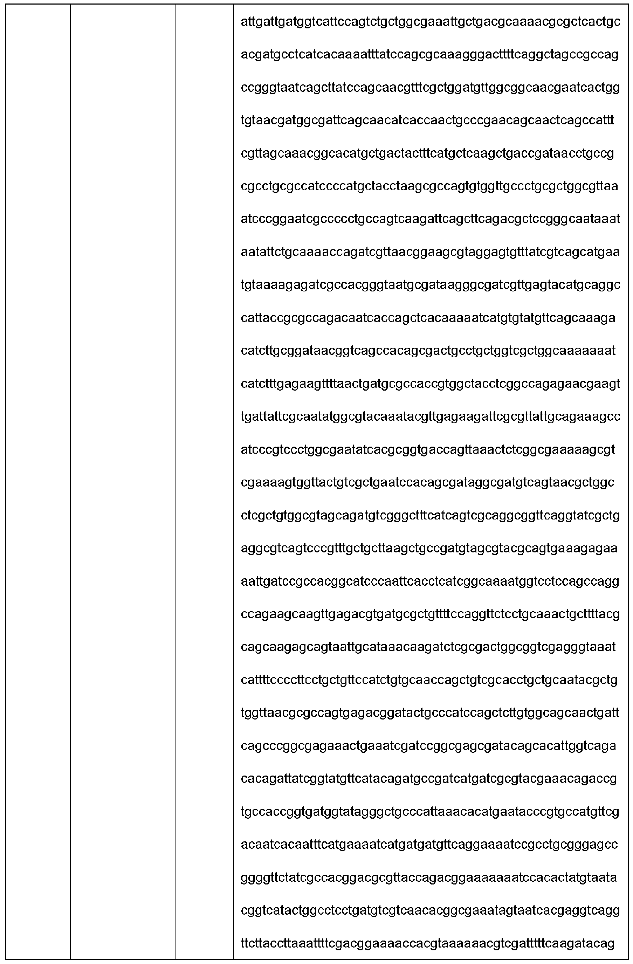

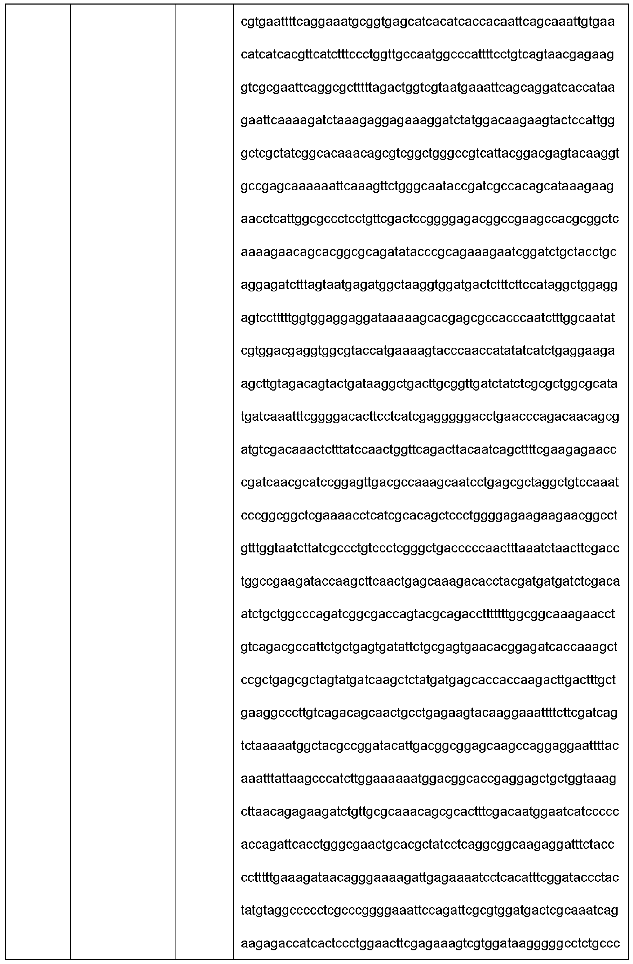

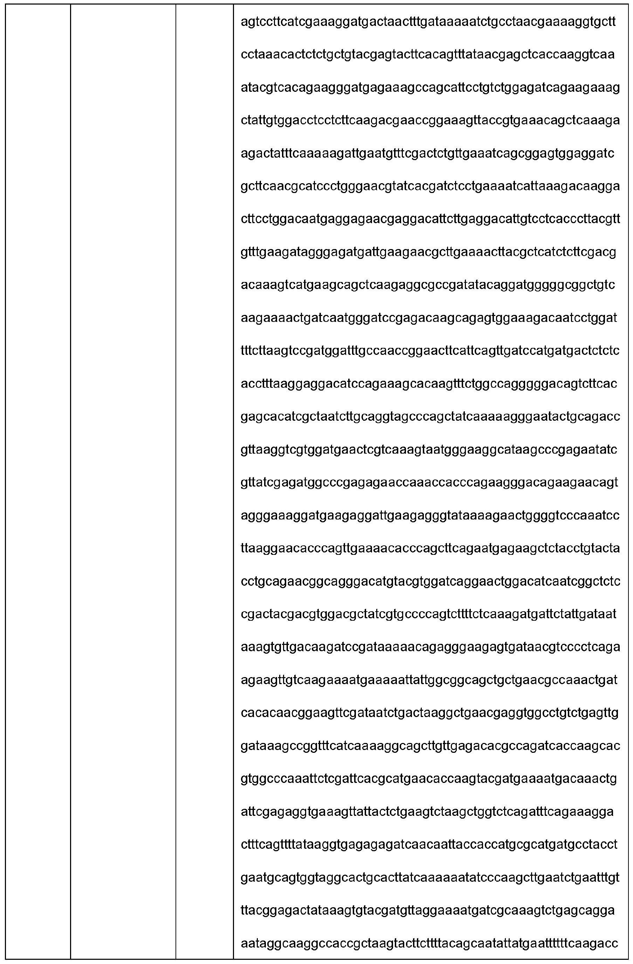

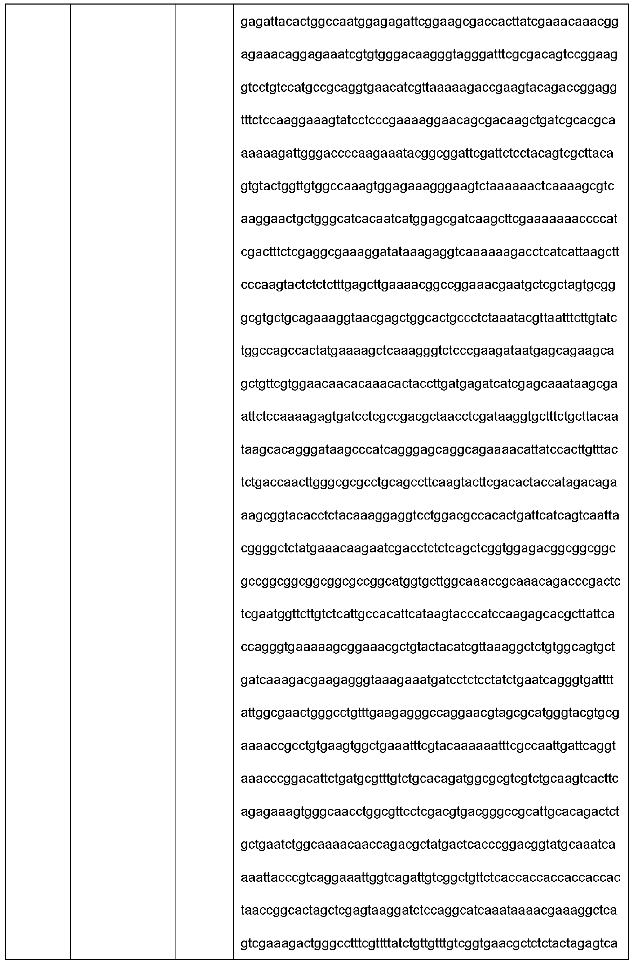

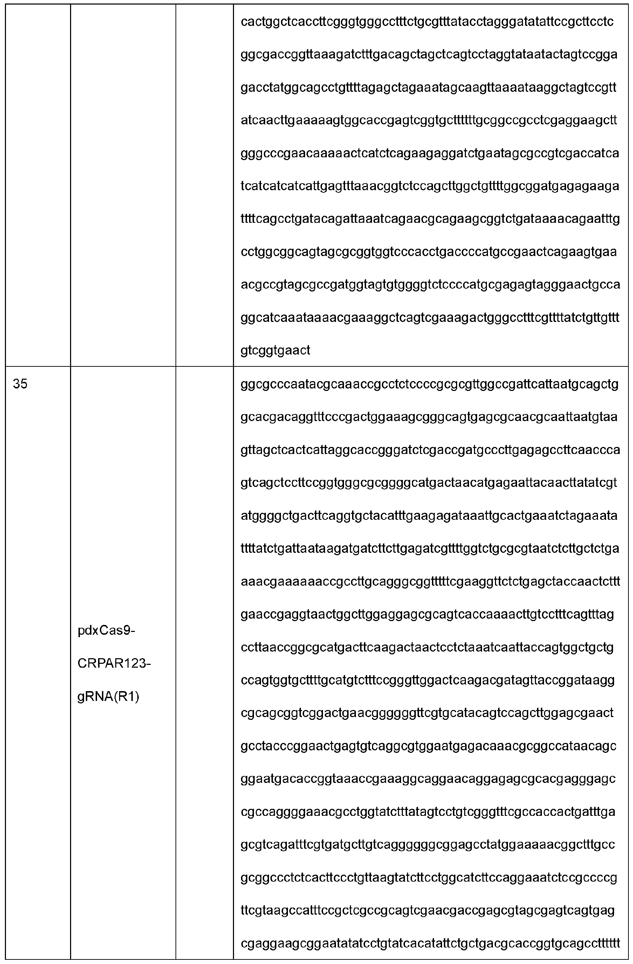

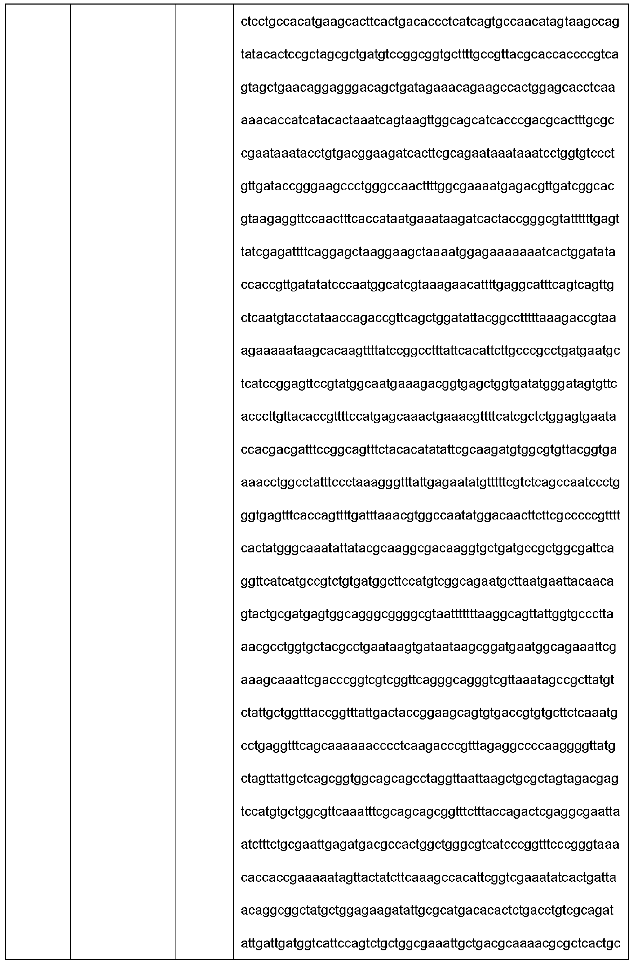

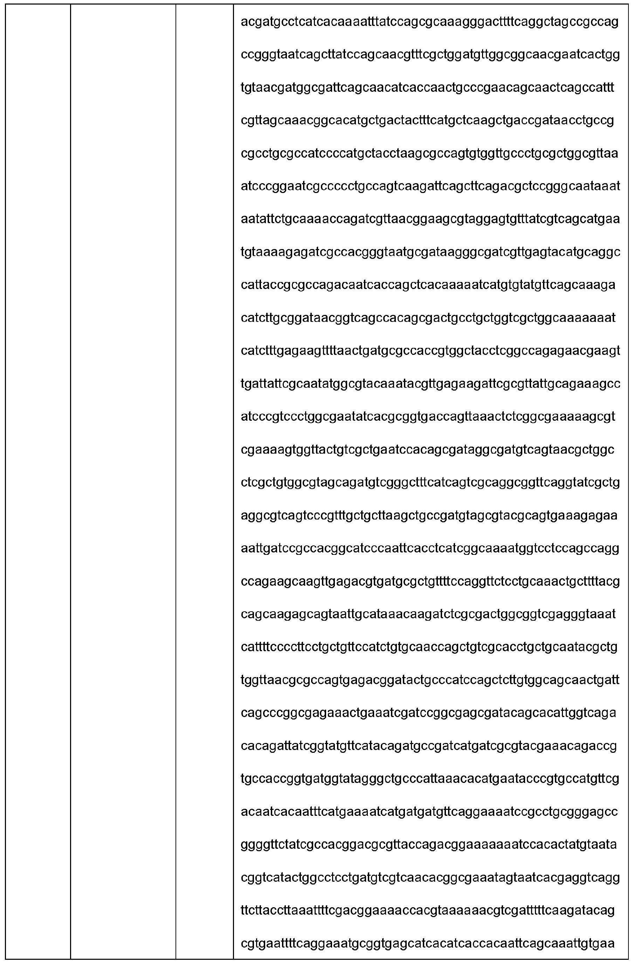

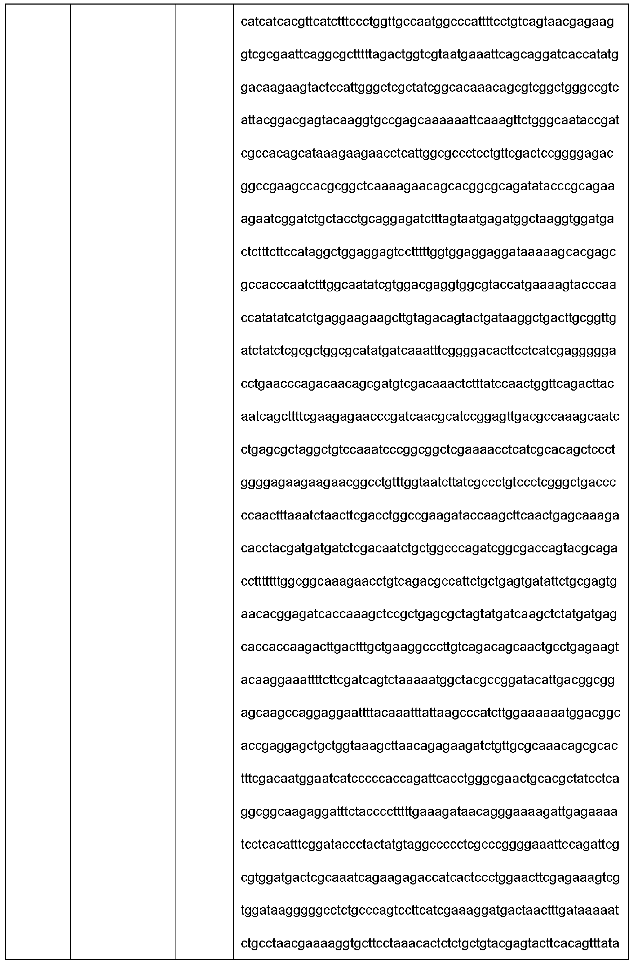

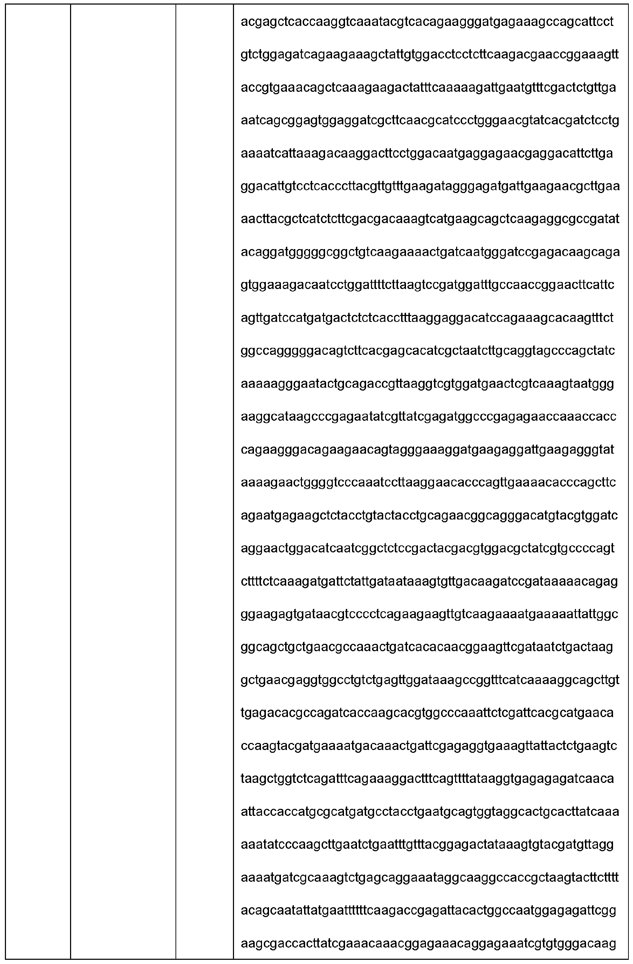

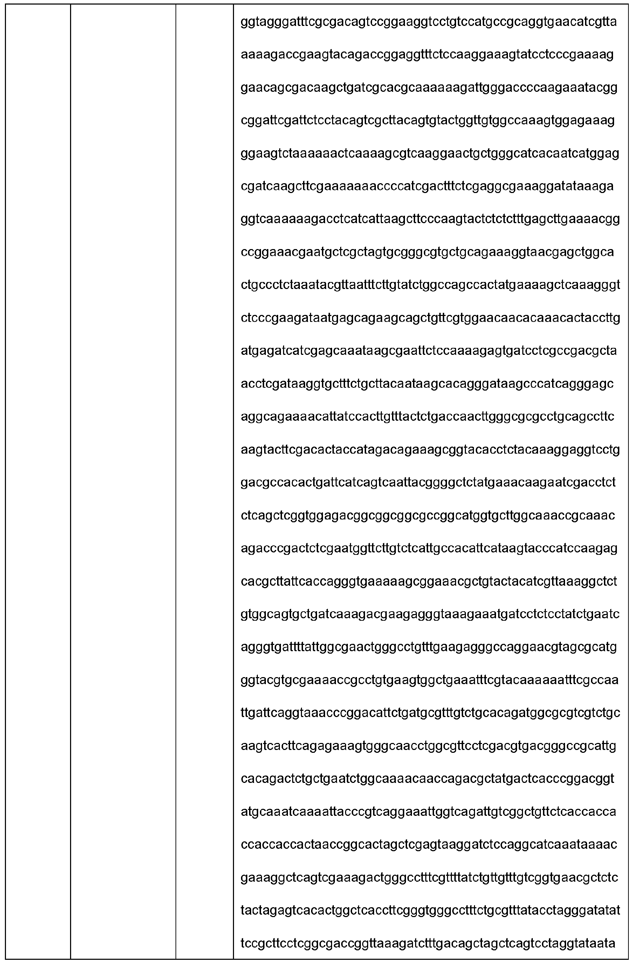

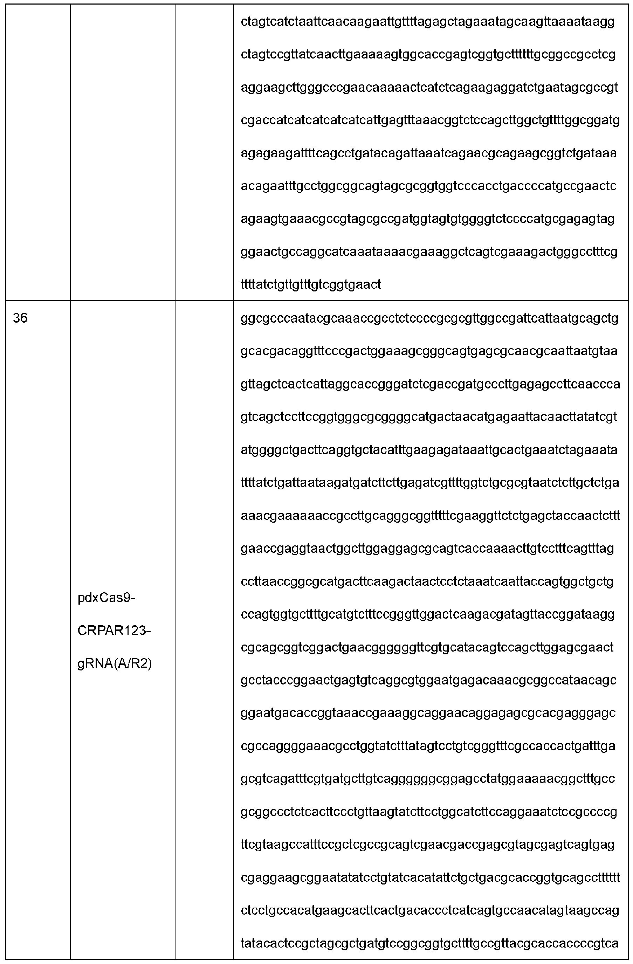

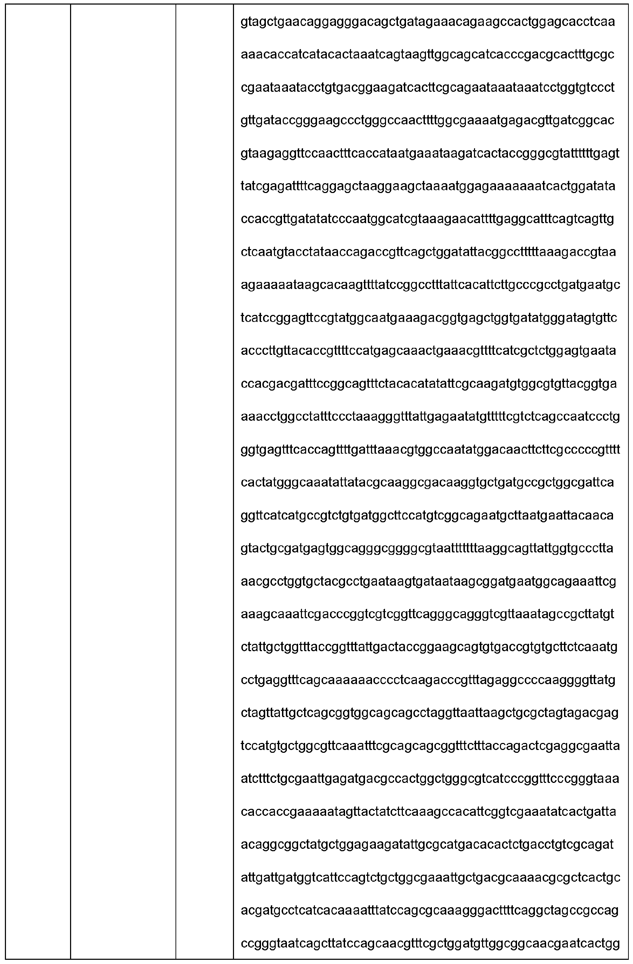

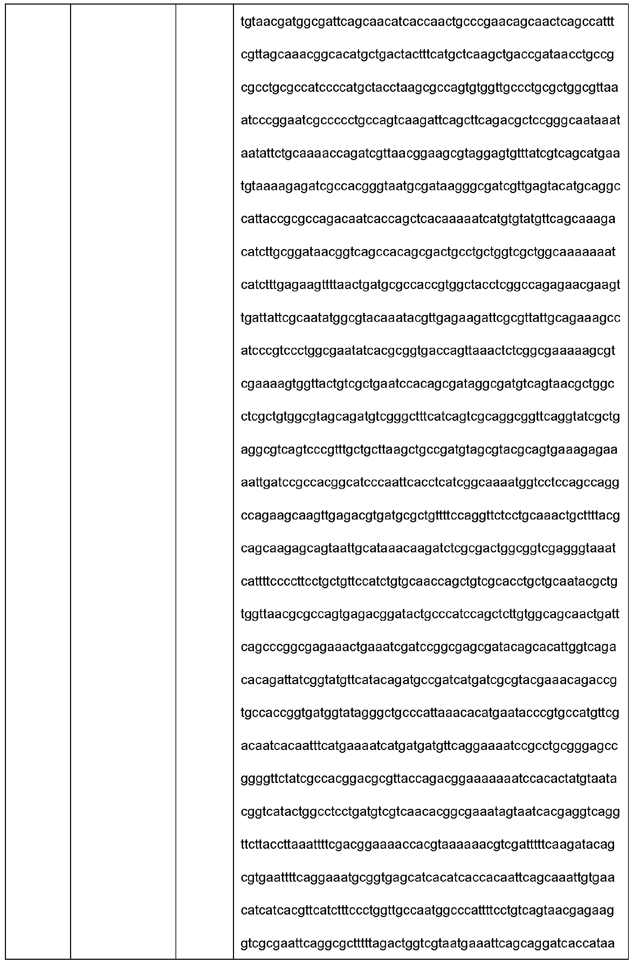

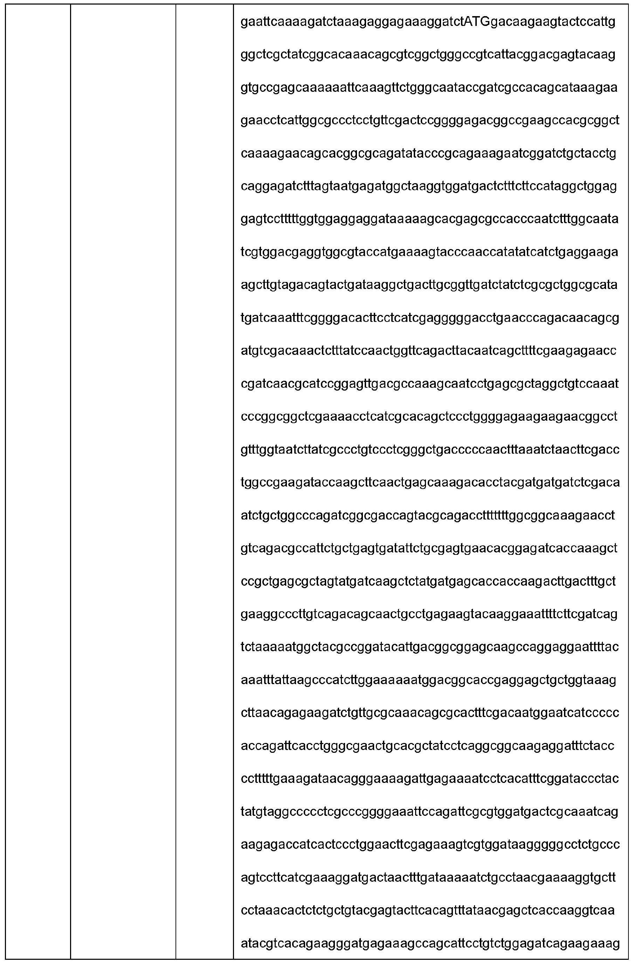

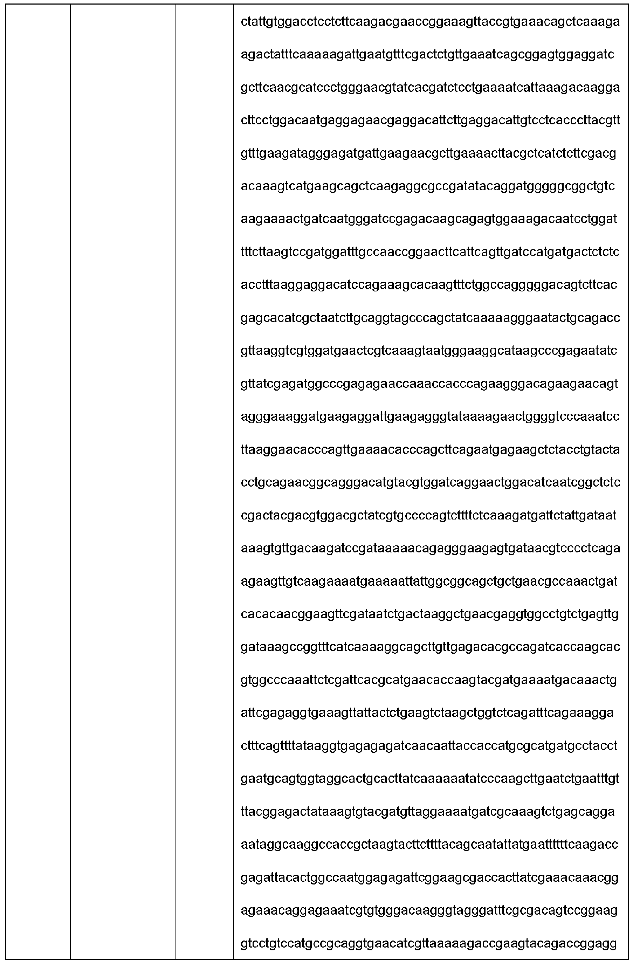

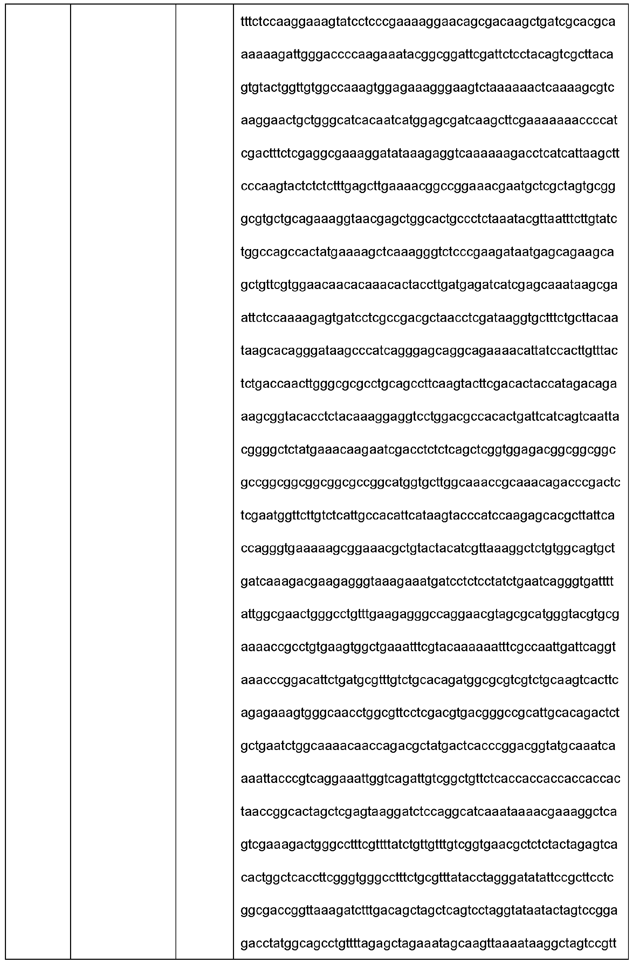

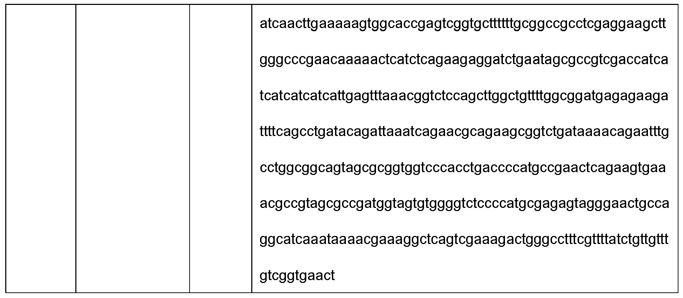

Additionally, as a result of verifying the effect of enhancing or inhibiting multiple gene expression, dxCas9-CRP_{AR123} showed an approximately 13-fold or higher increase in the expression level of GFP compared with a control strain and an approximately 93% or more reduction in the expression level of mCherry compared with control strains (FIG. 13).

The term "guide RNA (gRNA)" as used herein refers to an RNA that specifically binds to a target site, comprising the DNA nucleotide sequence of a target region, and guides dxCas9 to the corresponding target site.

Typically, the guide RNA may be a dual RNA consisting of two RNAs, that is, crRNA (CRISPR RNA) and trans-activating crRNA (tracrRNA), or may be in the form comprising a first region comprising a sequence that is fully or partially complementary to the sequence in the target DNA and a second region comprising a sequence that interacts with RNA-guide nuclease. As an example, when the guide RNA is applied to Cpf1, the guide RNA may be crRNA. As another example, when the guide RNA is applied to Cas9, the guide RNA may be in the form of dual RNA comprising crRNA and tracrRNA as constituent components or in the form of a single-chain guide RNA (sgRNA) in which main parts of crRNA and tracrRNA are fused.

In the present invention, gRNA may mean at least one gRNA selected from the group consisting of nucleotide sequences of SEQ ID NOS: 23 to 25, but is not limited thereto.

Herein, the expression having a specific sequence encompasses comprising, consisting essentially of, or consisting of the sequence represented by the specific sequence number. Herein, the sequence of the guide region has been described as one selected from the group consisting of nucleotide sequences of SEQ ID NOS: 23 to 25, but any sequence that has a modification, for example, addition, deletion, or substitution, in a part of the guide region may also fall within the scope of the guide sequence provided in the present invention as long as the sequence maintains the ability to bind complementarily to the target DNA sequence.

The term "plasmid" as used herein refers collectively to genes and extrachromosomal genes that exist in the cytoplasm of bacteria and are replicable. The plasmid of the present invention may comprise at least one gRNA comprising the target gene sequence and, specifically, may express at least one gRNA selected from the group consisting of nucleotide sequences of SEQ ID NOS: 23 to 25, but is not limited thereto.

In the present invention, although a DNA, RNA, polynucleotide, gene, or protein has been described as having a nucleotide sequence or amino acid sequence of a specific sequence number, it would be obvious that any DNA, RNA, polynucleotide, gene, or protein having a sequence with a deletion, modification, substitution, conservative substitution, or addition in a part may fall within the scope of the present invention as long as it has the same or corresponding activity with respect to the DNA, RNA, polynucleotide, gene, or protein consisting of the nucleotide sequence or amino acid sequence of the corresponding specific sequence number.

In addition, any sequence having at least 80% homology or identity with the nucleotide sequence or amino acid sequence represented by a specific sequence number may also fall within the scope of the present invention as long as it has the same or corresponding activity with respect to a DNA, RNA, polynucleotide, gene, or protein having the nucleotide sequence or amino acid sequence represented by the specific sequence number. Specifically, it would be obvious that any sequence that has at least 80%, 85%, 90%, 95%, 96%, 97%, 98%, or 99% homology or identity with the nucleotide sequence or amino acid sequence represented by the specific sequence number, exhibits corresponding efficacy or activity, and has a deletion, modification, substitution, or addition in a part of the sequence also falls within the scope of the present invention.

The term "homology" or "identity" as used herein refers to a degree of relevance between two given amino acid sequences or nucleotide sequences, and the term may be expressed as a percentage.

The terms homology and identity may be often used interchangeably with each other.

The sequence homology or identity of conserved polynucleotides or polypeptides may be determined by a standard alignment algorithm, and default gap penalties established by a program to be used may be used together. Substantially, homologous or identical sequences may be generally hybridized, under moderate or high stringent conditions, with the entire sequences or at least about 50%, 60%, 70%, 80%, or 90% of the full-lengths of the sequences. As for the hybridization, polynucleotides that contain degenerate codons instead of codons are also considered.

Whether any two polynucleotides or polypeptide sequences have homology, similarity, or identity may be determined using any computer algorithm known in the art, such as the "FASTA" program, using default parameters disclosed by Pearson et al (1988) [Proc. Natl. Acad. Sci. USA 85]: 2444. Alternatively, this may be determined using the Needleman-Wunsch algorithm (Needleman and Wunsch, 1970, J. Mol. Biol. 48: 443-453), which is performed in the Needleman program of the European Molecular Biology Open Software Suite (EMBOSS) package (Rice et al., 2000, Trends Genet. 16: 276-277) (version 5.0.0 or versions thereafter) (including the GCG program package (Devereux, J., et al., Nucleic Acids Research 12: 387 (1984)), BLASTP, BLASTN, FASTA (Atschul, [S.] [F.,] [ETAL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW.

The homology, similarity, or identity between polynucleotides or polypeptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358, 1979 (or the EDNAFULL (EMBOSS version of NCBI NUC4.4) substitution matrix); (2) a penalty of 3.0 for each gap and an additional 0.10 penalty for each symbol in each gap (or a gap opening penalty of 10 and a gap extension penalty of 0.5); and (3) no penalty for end gaps. Therefore, the term "homology" or "identity" as used herein represents the relevance between sequences.

The term "complementary" as used herein is used to describe the relationship between nucleotide bases that are capable of being hybridized with each other. For example, with respect to DNA, adenosine is complementary to thymine, and cytosine is complementary to guanine. Therefore, the present invention may also comprise substantially similar nucleic acid sequences as well as isolated nucleic acid fragments complementary to the entire sequence.

In accordance with another aspect of the present invention, there is provided a recombinant strain transformed with the plasmid.

In the present invention, the recombinant strain may control the gene expression, and the strain can enhance or inhibit the expression of a single target gene or multiple target genes, but is not limited thereto.

The "plasmid" and "target gene" are as described above.

The term "transformation" as used herein refers to a process of introducing a vector comprising a polynucleotide encoding a target protein into a host cell, thereby enabling the expression of the protein encoded by the polynucleotide in the host cell. A transformed polynucleotide may be inserted into the chromosome of the host cell and located thereon or located outside of the chromosome as long as the polynucleotide can be expressed in the host cell. In addition, the polynucleotide comprises DNA and RNA encoding the target protein. The polynucleotide may be introduced in any form as long as the polynucleotide can be introduced and expressed in a host cell. For example, the polynucleotide may be introduced into a host cell in the form of an expression cassette, which is a gene construct comprising all essential elements required for self-expression. The expression cassette may typically comprise a promoter, a transcription termination signal, a ribosome-binding domain, and a translation termination signal, which are operably linked to the polynucleotide. The expression cassette may be in the form of an expression vector capable of self-replication. In addition, the polynucleotide may be introduced in the form as it is into the host cell to be operably linked to the sequences required for expression in the host cell, but is not limited thereto.

Additionally, the term "operably linked" as used herein refers to a functional linkage between a promoter sequence, which initiates and mediates the transcription of the polynucleotide encoding the target protein of the present invention, and the gene sequence.

The method for transformation with the vector of the present invention may comprise any method by which a nucleic acid is introduced into a cell, and the transformation may be performed by selecting an appropriate standard technique, known in the art, according to the host cell. For example, examples of the method may comprise electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate/DMSO method, and the like, but are not limited thereto.

The "recombinant strain" as used herein refers to a strain into which a polynucleotide encoding a target recombinant protein is introduced in the form of an expression vector or in the form of being inserted into the chromosome so that the strain can express and produce the target recombinant protein. Specifically, the recombinant strain in the present invention may mean a strain that enhances and/or inhibits not only a single target gene of the strain but also multiple genes by using dxCas9-CRP or a dxCas9-CRP derivative, but is not limited thereto.

The strain in the present invention may belong to the genus *Escherichia,* and specifically may be *Escherichia coli,* but is not limited thereto.

In accordance with still another aspect of the present invention, there is provided a system for controlling the expression of a target gene, the system comprising dxCas9 and a CRP derivative.

The "dxCas9", "CRP derivative", "target gene", and "controlling the expression" are as described above.

The term system for controlling the expression of a target gene may mean that the expression of a single target gene can be enhanced or inhibited, or in case of multiple genes, some genes can be enhanced and other genes can be inhibited, that is, the simultaneous enhancement and inhibition of the expression of multiple genes can be achieved, but is not limited thereto.

In an embodiment of the present invention, the GFP fluorescence expression of the target gene gRNA(A) was increased compared with a control and the mCherry fluorescence expression of the target gene gRNA(R2) was reduced compared with a control. Therefore, the system of the present invention can enhance the expression of some genes and inhibit the expression of other genes, indicating that the system of the present invention can achieve simultaneous control of the enhancement and inhibition of genes (FIG. 13).

The present invention has technical significance in developing a system for controlling the expression of a target gene that can perform the functions of transcriptional activation and repression of a single gene or multiple genes.

In accordance with still another aspect of the present invention, there is provided a method for preparing a recombinant strain controlling the expression of a target gene, the method comprising: i) constructing a dxCas9-CRP system in which a dxCas9 protein is bound to a CRP protein; ii) cloning a fluorescent reporter plasmid in the constructed dxCas9-CRP system; iii) additionally cloning a guide RNA in the dxCas9-CRP system constructed in step ii); and iv) transforming the dxCas9-CRP-gRNA into a strain.

In the present invention, the guide RNA may comprise a target gene.

In the present invention, the dxCas9 may be bound to a linker comprising the amino acid sequence of SEQ ID NO: 2, but is not limited thereto.

In the present invention, the strain may belong to the genus *Escherichia,* and specifically may be *Escherichia coli,* but is not limited thereto.

The "dxCas9", "CRP", "system", "guide RNA", "transformation", and "recombinant strain" are as described above.

In the present invention, the fluorescent reporter may be used to verify the performance of the dxCas9-CRP system. The fluorescent reporter may be, for example, luciferase, β-galactosidase, green fluorescent protein (GFP), enhanced green fluorescent protein (eGFP), mPlum, mCherry, tdTomato, mStrawberry, J-Red, DsRed, mOrange, mKO, mCitrine, Venus, YPet, enhanced yellow fluorescent protein (EYFP), Emerald, CyPet, cyan fluorescent protein (CFP), cerulean, T-Sapphire, and alkaline phosphatase and, specifically, may be GFP or mCherry, but is not limited thereto.

In accordance with still another aspect of the present invention, there is provided a method for controlling the expression of a target gene, the method comprising applying a CRP derivative to dxCas9.

The "dxCas9", "CRP", "target gene", and "expression regulation" are as described above.

### Advantageous Effects

The plasmid, recombinant strain, and expression control system according to the present invention enable the construction of a system controlling the expression of target genes simultaneously and multiply, thereby improving the production of high-value materials.

### Brief Description of Drawings

FIG. 1 shows design diagrams of a plasmid for a CRISPR-based gene expression control system and a GFP fluorescence expression plasmid for validating system performance.
FIG. 2 shows a schematic diagram of enhancement/inhibition control of target gene expression by the CRISPR-based gene expression control system.
FIG. 3 shows a map of pMW7-P_{J23117}-GFP plasmid.
FIG. 4 shows a map of pMW7-P_{J23119}-GFP plasmid.
FIG. 5 shows a map of pMW7-P_{J23117}-GFP-P_{J23119}-mCherry plasmid.
FIG. 6 shows the gRNA target positions on the fluorescent plasmid (target gene).
FIG. 7 shows a cloning design for gRNA expression.
FIG. 8 shows a map of pdxCas9-CRP_{AR123}-gRNA(A) plasmid.
FIG. 9 shows a map of pdxCas9-CRP_{AR123}-gRNA(R1) plasmid.
FIG. 10 shows a map of pdxCas9-CRP_{AR123}-gRNA(A/R2) plasmid.
FIG. 11 shows a graph comparing target gene transcriptional activation efficiency among dxCas9-binding CRP derivatives.
FIG. 12 shows a graph comparing target gene transcriptional repression efficiency among dxCas9-binding CRP derivatives.
FIG. 13 shows a diagram of simultaneous expression enhancement/repression control of GFP/mCherry genes by pdxCas9-CRP_{AR123} and a graph of validation results of multiple target possibility of a dxCas9-CRP_{AR123} system.

### Detailed Description of the Invention

Hereinafter, the present invention will be described in detail with reference to examples and experimental examples. However, these examples and experimental examples are given for specifically illustrating the present invention, and the scope of the present invention is not limited thereto.

### Experimental Example 1. Construction of CRISPR-based gene expression control dxCas9-CRP_{AR123} system

### Experimental Example 1-1. Preparation of pdxCas9-linker

The overexpression of dxCas9 may cause cytotoxicity, and thus the dxCas9 gene was inserted into the pAR-mlacI [rhaPBAD, p15A ori, CmR] vector to introduce an L-Rhamnose inducible promoter system capable of precise expression control.

Specifically, for the preparation of a dxCas9-linker composed of dxCas9 (SEQ ID NO: 1) and a linker (SEQ ID NO: 2), dxCas9-linker DNA was amplified by PCR using the dxCas9(3.7)-VPR (Addgene plasmid #108383) plasmid as a template along with the primers of SEQ ID NOS: 3 and 4. The primer sequences used in the experiment are shown in Table 1 below.

**TABLE 1**

| **Primer name** | **Primer sequence (5'→ 3')** | **SEQ ID NO** |
|---|---|---|
| dxCas9_*RBS*_Ndel_F | agcaggatcaccataagaattcaaaagatctaaagaggagaaaggatc tatggacaagaagtact | 3 |
| dxCas9-linker_Spel_R | ctagactagtgccggcgccgccgccgtctccaccgagctgagagag | 4 |

Next, the pdxCas9-linker plasmid was prepared by homologous recombination of the pAR-mlacI vector and dxCas9-linker PCR products, cleaved with *Ndel* and Spel restriction enzymes, and then the prepared recombinant plasmid was transformed into *E. coli* DH5α strain by heat shock. The transformed strain was cultured on solid-LB media containing 25 µg/ml of the antibiotic chloramphenicol, and then cells with the recombinant plasmid inserted were selected by PCR using the primers (SEQ ID NOS: 3 and 4). Thereafter, the plasmid was extracted from the selected cells to finally obtain the recombinant pdxCas9-linker plasmid.

### Experimental Example 1-2. Preparation of dxCas9 and CRP (WT and derivative) binding protein expression plasmids

CRP is structurally composed of AR1, AR2, and AR3 (activating regions), which bind to the α-subunit of RNA polymerase to activate gene transcription, and binds to the CRP regulon gene through a DNA-binding motif present at the C-terminus. To select a domain that functions as an effector by binding to dxCas9 and exhibits the maximum effect, among the domains of CRP, WT CRP (wild type, CRP_{WT}, SEQ ID NO: 5) of *E. coli* and four CRP derivatives CRP_{AR123} (SEQ ID NO: 6), CRP_{AR23} (SEQ ID NO: 7), CRP_{AR1} (SEQ ID NO: 8), and CRP_{AR3} (SEQ ID NO: 9) were selected as effector domains and investigated for gene expression control effects.

Specifically, CRP_{WT} was a wild-type transcriptional regulator of 45 kDa consisting of a total of 210 amino acids, with AR2 (20th, 22nd, 97th, and 102nd residues) and AR3 (53rd to 56th and 59th residues) at the N-terminus, which are activating regions capable of binding to the α-subunit of RNA polymerase, and AR1 (157th to 165th residues) and a DNA-binding motif (182nd to 194th residues) at the C-terminus, which recognizes and binds to the CRP regulon gene, wherein the N-terminus and C-terminus are linked by a short hinge region (136th to 139th residues). CRP_{AR123} consisted of a total of 180 amino acids of 1st to 180th amino acids and comprised all of the activating region (AR1, AR2, AR3) domains, with a deletion of the DNA-binding motif at the C-terminus in the above-described wild-type CRP_{WT}. CRP_{AR23} consisted of a total of 139 amino acids of 1st to 139th amino acids and comprised AR2 and AR3 domains and the hinge region at the N-terminus, with a deletion of the entire C-terminus in the wild-type CRP_{WT}. CRP_{AR1} consisted of a total of 45 amino acids of 136th to 180th amino acids and comprised only the AR1 domain at the C-terminus in the wild-type CRP. Lastly, CRP_{AR3} consisted of a total of 60 amino acids of 28th to 92nd amino acids and comprised only AR3 at the N-terminus in the wild-type CRP_{WT}. These were selected and experimented as follows.

To insert the WT CRP and CRP derivatives into the pdxCas9-linker plasmids prepared in Experimental Example 1-1 above, respectively, primers were designed to partially express each domain of CRP by using the E. *coli* MG1655 (DE3) genome as a template. PCR was performed by using the primers of SEQ ID NOS: 10 and 13 for CRP_{WT}, the primers of SEQ ID NOS: 10 and 14 for CRP_{AR123}, the primers of SEQ ID NOS: 10 and 15 for CRP_{AR23}, the primers of SEQ ID NOS: 11 and 14 for CRP_{AR1}, and the primers of SEQ ID NOS: 12 and 16 for CRP_{AR3}, and thereafter, each PCR product was obtained. The primer sequences used in the experiment are shown in Table 2 below.

**TABLE 2**

| **Primer name** | **Primer sequence (5'→ 3')** | **SEQ ID NO** |
|---|---|---|
| CRP_NgoMIV_F | cggcggcggcgccggcatggtgcttggcaaaccg | 10 |
| CRP-AR1-NgoMIV-F | cggcggcggcgccggcgcgttcctcgacgtgacgg | 11 |
| CRP-AR3-NgoMIV-F | cggcggcggcgccggcagcacgcttattcaccaggg | 12 |
| CRP-His6-NgoMIV-R | gaggactagtgccggttagtggtggtggtggtggtgacgagtgccgtaaacgac ga | 13 |
| CRP-AR123-NgoMIV-R | gaggactagtgccggttagtggtggtggtggtgagaacagccgacaatctg acca | 14 |
| CRP-AR23-NgoMIV-R | gaggactagtgccggttagtggtggtggtggtggtggtcgaggaacgccaggttg | 15 |
| CRP-AR3-NgoMIV-R | gaggactagtgccggttagtggtggtggtggtggtggtgggcggttttcgcacgtacc | 16 |

Next, the dxCas9-linker plasmid treated with *NgoMIV* restriction enzymes and the amplified PCR products of the CRP derivatives (CRP_{AR123}, CRP_{AR23}, CRP_{AR1}, and CRP_{AR3}) were subjected to homologeneous recombination to prepare pdxCas9-CRP_{WT}, pdxCas9-CRP_{AR123}, pdxCas9-CRP_{AR23}, pdxCas9-CRP_{AR1}, and pdxCas9-CRP_{AR3} recombinant plasmids, respectively. Then, the recombinant reaction products were used for transformation by the same method as in Experimental Example 1-1 above, and then recombinant plasmids were selected by using the respective primers (SEQ ID NOS: 10 to 16) used in the amplification of PCR products of the recombinant plasmids of the WT CRP and respective CRP derivatives.

### Experimental Example 2. Preparation of fluorescent reporter plasmid for validation of dxCas9-CRP_{AR123} system

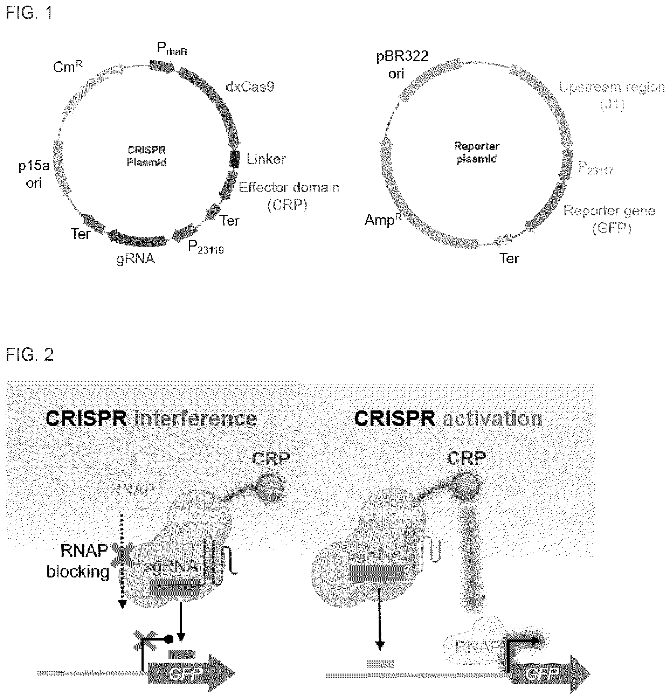

To test and validate the transcriptional regulation (activation/repression) performance of dxCas9-CRP systems (FIGS. 1 and 2), the GFP fluorescent gene and the mCherry fluorescent gene, of which the expression levels can be easily measured, were selected as reporter genes.

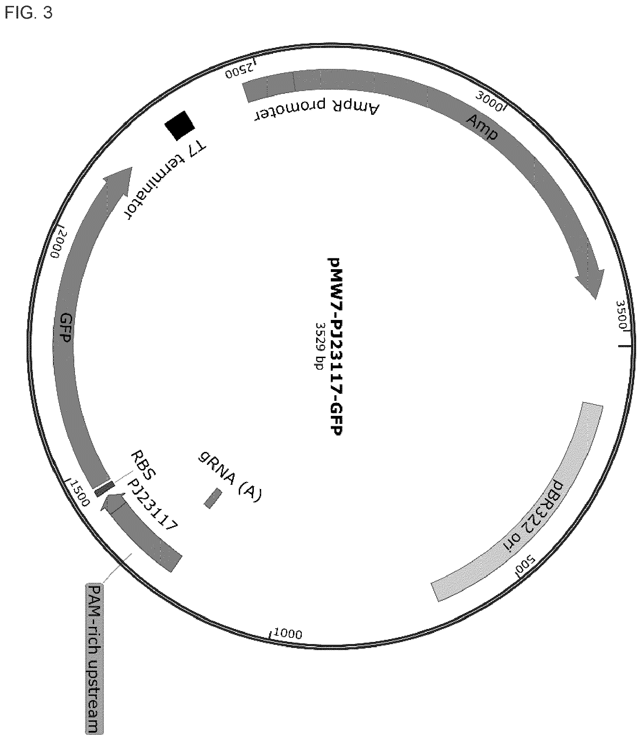

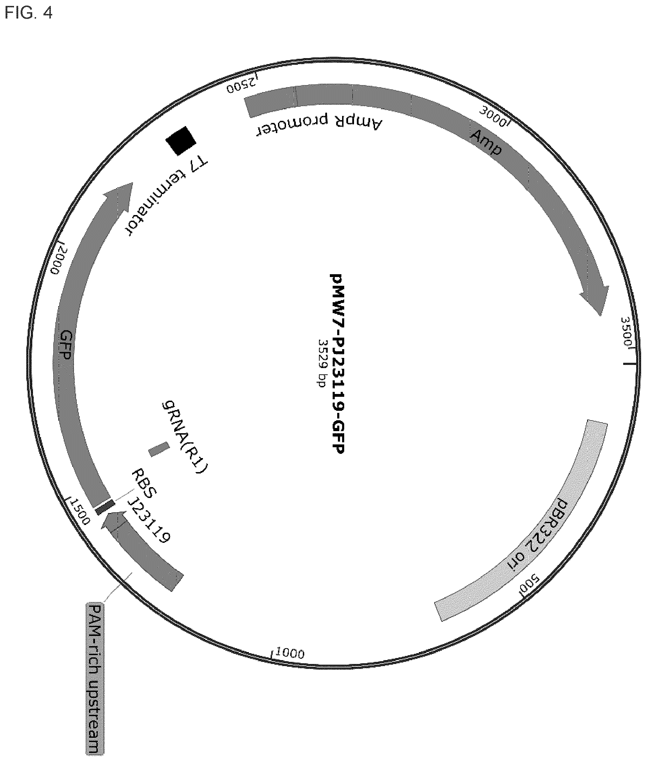

To validate whether transcriptional activation occurred or not, the linkage sequence of the gene sequence J1 of 170 bp (Cheng dong et al., 2018, Nature Communications, 9:2489), which is a PAM-rich upstream containing an NGG sequence, and P_{J23119}/or P_{J23117}, and *RBS* was obtained by gene synthesis since dxCas9-CRP recognized and bound to the upstream region of a target gene. PCR was performed using the obtained synthetic gene as a template for PCR reaction along with the primers of SEQ ID NOS: 17 and 18 for J1-P_{J23119}-*RBS* and the primers of SEQ ID NOS: 19 and 20 for *J1*-P_{J23117}-*RBS*. The pMW7-GFP plasmid cleaved with *Ndel* restriction enzymes and the obtained PCR products were subjected to homologous recombination to prepare the recombinant plasmids pMW7-P_{J23117}-GFP (weak GFP expression) and pMW7-P_{J23119}-GFP (strong GFP expression), of which the GFP expression intensity was artificially controlled by a synthetic promoter (FIGS. 3 and 4).

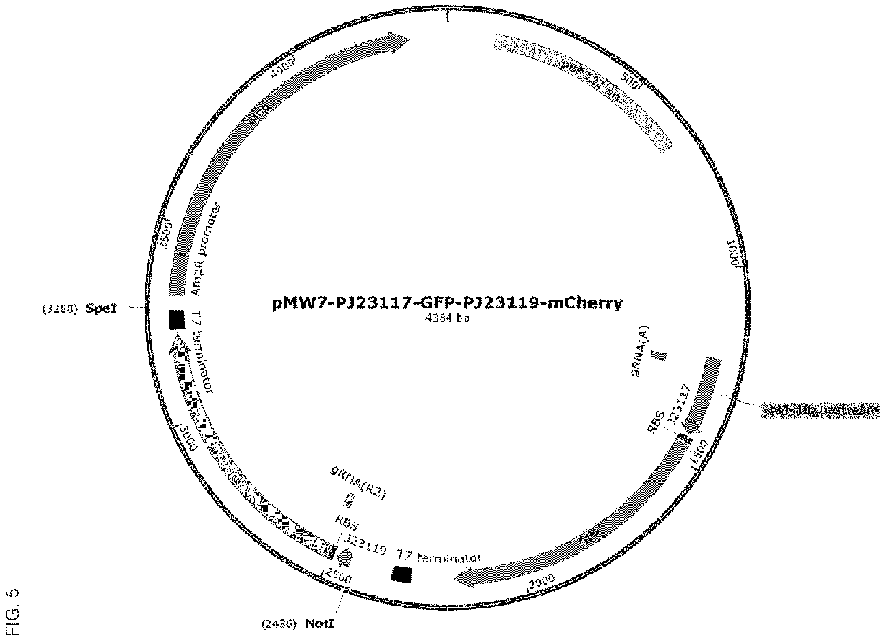

In addition, to validate whether a dxCas9-CRP system simultaneously regulates transcriptional activation and repression of multiple genes in one cell, the pMW7-P_{J23117}-GFP-P_{J23119}-mCherry plasmid consistently inducing weak GFP expression and strong mCherry expression was prepared by artificially controlling the expression intensity of the fluorescent genes through a synthetic promoter. The pMW7-P_{J23117}-GFP-P_{J23119}-mCherry plasmid was prepared by homologous recombination of a PCR product of P_{J23119}-mCherry amplified by PCR performed using the primers of SEQ ID NOS: 21 and 22 and the pMW7-P_{J23H7}-GFP plasmid cleaved with *AatII* restriction enzymes (FIG. 5). The plasmid with P_{J23119}-mCherry inserted was selected by PCR using the primers of SEQ ID NOS: 21 and 22.

The primer sequences used in the preparation of the fluorescent reporter plasmids are shown in Table 4 below.

**TABLE 4**

| **Primer name** | **Primer sequence (5'→ 3')** | **SEQ ID NO** |
|---|---|---|
| ***J1*-P_{J23119}-*RBS* amplification** | | |
| J1-Ndel-F | aaggagatatacatatggcctacggtatccaccgg | 17 |
| J1-Ndel-R | tcttctcctttactcatatgacctttctcctctttaatgaat | 18 |

| ***J1*-P_{J23117}-*RBS* amplification** | | |
|---|---|---|
| J1-Ndel-F | aaggagatatacatatggcctacggtatccaccgg | 19 |
| _{J23117}-Ndel-Infu-R | tcttctcctttactcatatgacctttctcctctttaatgaattcgctagcacaatcc ctaggactgagctagctgtcaagtcc | 20 |

| **P_{J23119}-*mCherry* amplification** | | |
|---|---|---|
| _{J23119}_mCherry_BamHI_F | gggaaatgtgggatcgccggcttgacagctagctcagtcctaggtataatg ctagcgaattcattaaagaggagaaaggtaccatggtaagtaagggtga ag | 21 |
| mCherry_BamHI_R | tggcgatatcggatccaaaaaacccctcaagacccgtttagaggcccca aggggttatgctagcccgggagatctttatttgtacaattcgtccattc | 22 |

### Experimental Example 3. Selection of target positions for fluorescent gene expression enhancement and inhibition and gRNA preparation

To allow dxCas9 to recognize and bind to the fluorescent gene of the fluorescent reporter plasmid prepared in Example 2 above, the gRNA target positions on the gene were selected, and cloning was performed for additional gRNA expression in each of the pdxCas9-linker-CRP (or CRP derivative) plasmids prepared in Experimental Example 1-2 above.

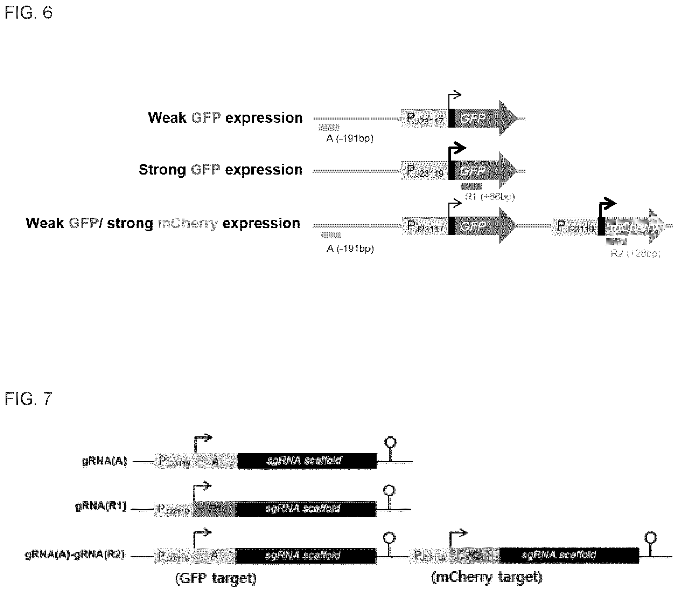

Specifically, gRNA comprises a target gene sequence, and the transcriptional activation or repression of the gene is determined according to the binding position of gRNA to the target gene, and thus the binding positions, adjacent to the PAM (5'-NGG-3') sequence, of gRAN(A) (SEQ ID NO: 23) for GFP expression activation located at - 191 bp and gRNA(R1) (SEQ ID NO: 24) for GFP expression repression located at +66 bp from the transcription start point were selected, and the target position of gRNA(R2) (SEQ ID NO: 25) for mCherry expression repression located at +28 bp from the transcription start point was selected (FIG. 6). The gRNA sequence information used herein is shown in Table 5 below.

**TABLE 5**

| **gRNA name** | **gRNA sequence** | **Target gene** | **Target position (based on transcription start point)** | **SEQ ID NO** |
|---|---|---|---|---|
| gRNA(A) | ccggagacctatggcagcct | GFP | -191 bp | 23 |
| gRNA(R1) | catctaattcaacaagaatt | GFP | +66 bp | 24 |
| pRNA(R2) | ttcttcacccttacttacca | mCherry | +28 bp | 25 |

Then, gRNA is expressed to act as a structure in which the dxCas9 handle sequence with a 42-bp hairpin structure capable of binding to dxCas9 and the sgRNA scaffold consisting of a 40-bp terminator derived from *Streptococcus pyogenes* strain are linked together with a sequence of 20 bp complementarily binding to the target gene. All the gRNAs were induced to overexpress by the introduction of P_{J23119} with consistent strong expression intensity (FIG. 7).

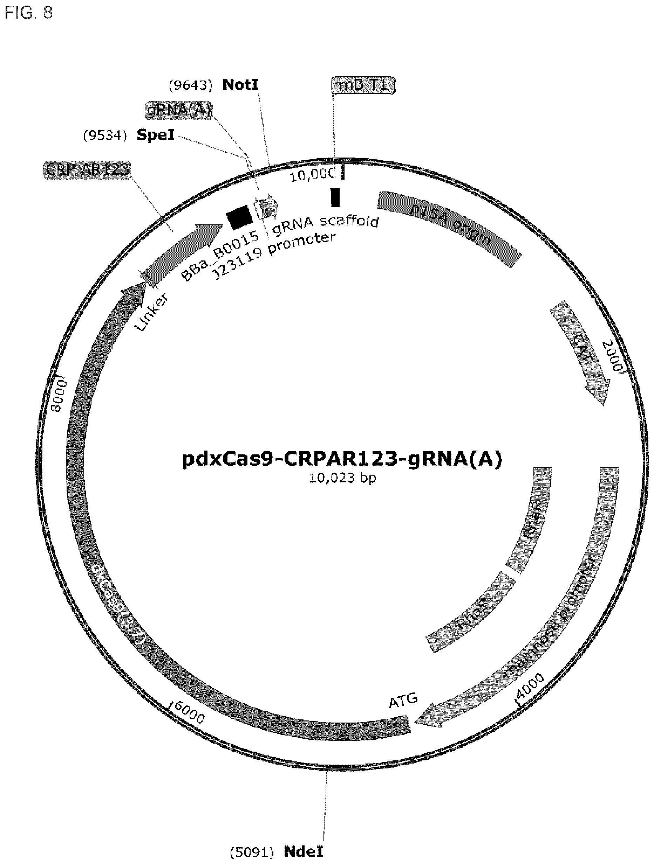

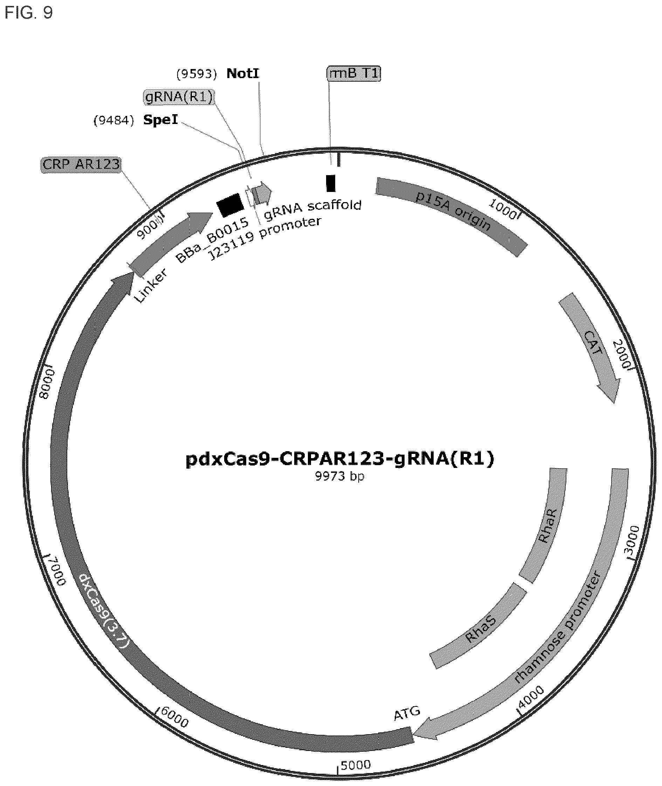

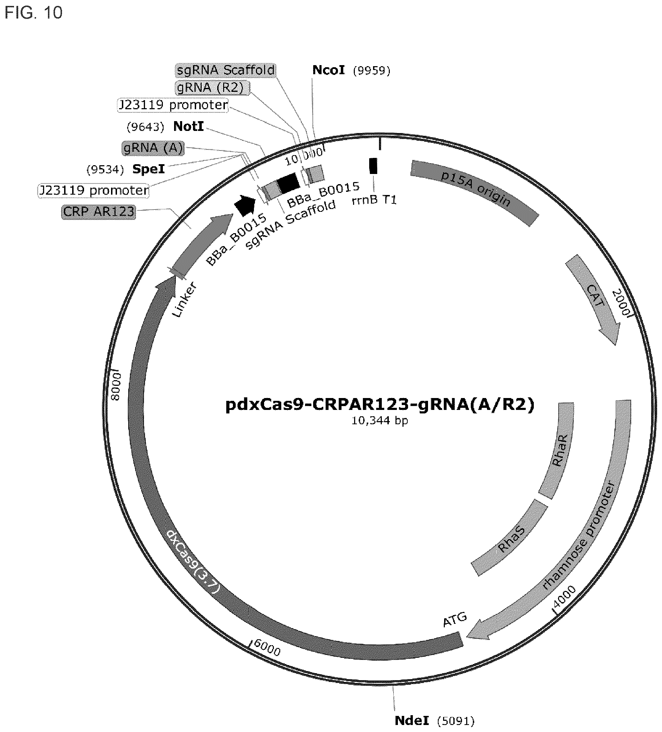

To prepare the pdxCas9-CRP-gRNA plasmid for expressing each gRNA, PCR was first performed, for amplification of gRNA(A), gRNA(R1), and gRNA(R2), by using pgRNA-bacteria (Addgene #44251) as a template along with a primer combination of SEQ ID NOS: 26 and 27 and a primer combination of SEQ ID NOS: 28 and 33 for gRNA(A), a primer combination of SEQ ID NOS: 26 and 29 and a primer combination of SEQ ID NOS: 30 and 33 for gRNA(R1), and a primer combination of SEQ ID NOS: 26 and 31 and a primer combination of SEQ ID NOS: 32 and 33 for gRNA(R2), thereby obtaining two types of PCR products for each. Overlap PCR based on an overlapping sequence of the two types of PCR products obtained was performed to obtain PCR products of gRNA(A), gRNA(R1), and gRNA(R2) comprising the target gene sequence. Next, the pdCas9-CRP (or CRP derivative) plasmid prepared in Experimental Example 1-2 above was subjected to homologous recombination together with linear DNA cleaved with Spel and *Notl* restriction enzymes and gRNA(A) or gRNA(R1) PCR products, thereby preparing recombinant pdxCas9-CRP (or CRP derivative)-gRNA(A) plasmid (FIG. 8, SEQ ID NO. 34) or pdxCas9-CRP (or CRP derivative)-gRNA(R1) plasmid (FIG. 9, SEQ ID NO. 35). The transformation with the recombinant reaction products and the selection of the recombinant plasmids were carried out by the same method as in Experimental Example 1-1 above, and the recombinant plasmids were selected by performing PCR with the primers of SEQ ID NO: 26 and 33. The linear pdxCas9-CRP-gRNA(A) plasmid cleaved with *Notl* restriction enzyme and the PCR product of gRNA(R2) were subjected to homologous recombination to prepare a recombinant plasmid in which gRNA(R2) was additionally inserted into pdxCas9-CRP-gRNA(A). Thereafter, the prepared plasmid was cleaved with *Notl* and *Ncol* restriction enzymes, subjected to agarose gel electrophoresis, and a plasmid detected to show a DNA band corresponding to the size of gRNA(R2) was selected to produce pdxCas9-CRP-gRNA (A/R2) plasmid (FIG. 10, SEQ ID NO: 36).

The primer sequences used in the experiment are shown in Table 6 below.

**TABLE 6**

| **Primer name** | **Primer sequence (5'→ 3')** | **SEQ ID NO** |
|---|---|---|
| **gRNA amplification** | | |
| Ter-sgRNA_Spel_F | Actaaccggcactagctcgagtaaggatctccaggcatc | 26 |
| Ter_A_gRNA_R | Aggctgccataggtctccggactagtattatacctaggactgagctagctg tcaa | 27 |
| A_gRNA_2F | Taatactagtccggagacctatggcagcctgttttagagctagaaatagca agtt | 28 |
| Ter_R1_gRNA_R | Aattcttgttgaattagatgactagtattatacctaggactgagctagctgtca a | 29 |
| R1_gRNA 2F | Taatactagtcatctaattcaacaagaattgttttagagctagaaatagcaa gtt | 30 |
| Ter_R2_gRNA_R | Tggtaagtaagggtgaagaagctagcattatacctaggactgagctagct gtcaa | 31 |
| R2_gRNA 2F | Taatgctagcttcttcacccttacttaccagttttagagctagaaatagcaag tt | 32 |
| Ter-sgRNA_NotI-R | ttcctcgaggcggccgcaaaaaagcaccgactcggtgccactt | 33 |

**TABLE 7**

| **Plasmid Name** | **Plasmid description** | **SEQ ID NO** |
|---|---|---|
| pdxCas9-CRP_{AR123}-gRNA(A) | *rhaP_{BAD}::dxCas9-linker-CRP_{AR123}, sgRNA (A), p15A ori, Cm^{R}* | 34 |
| pdxCas9-CRP_{AR123}-gRNA(R1) | *rhaP_{BAD}::dxCas9-linker-CRP_{AR123}, sgRNA (R1), p15A ori, Cm^{R}* | 35 |
| pdxCas9-CRP_{AR123}-gRNA(A/R2) | *rhaP_{BAD}::dxCas9-linker-CRP_{AR123}, sgRNA (A), sgRNA(R2), p15A ori, Cm^{R}* | 36 |

### Example 1. Validation of gene expression enhancement/inhibition control of dxCas9-CRP system

### Example 1-1. Verification of single target gene expression enhancing effect of dxCas9-CRP and selection of CRP derivative

The recombinant pdxCas9-CRP (or CRP derivative)-gRNA(A) plasmid constructed in Experimental Example 3 and the GFP reporter plasmid constructed in Experimental Example 2 were transformed together into the strain E. *coli* MG1655 by using a heat shock method, and then the strain was cultured on solid LB medium containing 25 µg/ml of chloramphenicol and 100 µg/ml of ampicillin, thereby selecting transformed strains. Then, the selected strains were cultured in liquid-LB medium containing 1 mM L-Rhamnose, and the cells cultured for 24 hours were imaged with a confocal fluorescence microscope. Thereafter, the GFP expression levels of individual cells were measured by fluorescence intensity (FIG. 11).

As a result, as can be confirmed in FIG. 11, the control strains expressing dxCas9 and non-targeting gRNA expressed relatively low levels of GFP fluorescence intensity, and the dxCas9-CRP_{AR123} derivative expressing strain showed an approximate 4-fold or higher increase in GFP fluorescence expression level compared with the control strains and showed the highest fluorescence expression compared with the other CRP derivative expressing strains.

Therefore, it was verified that the CRP_{AR123} derivative had the greatest transcriptional enhancement effect as an effector of dxCas9, and the CRP_{AR123} derivative contained all of AR1, AR2, and AR3, which are activating regions capable of binding to RNA polymerase due to the protein structure thereof, and the CRP_{AR123} derivative showing excellent transcriptional enhancement control activity was selected as the most suitable CRP derivative, and then experiments were carried out.

### Example 1-2. Verification of single target gene expression inhibiting effect of dxCas9-CRP and selection of CRP derivative

The binding of the dxCas9 protein to the promoter and ORF site of a target gene interferes with the access of RNA polymerase to repress the transcription with high efficiency, and thus an experiment was carried out to investigate, through the infusion of dxCas9 and CRP (or CRP derivative), the influence of dxCas9 alone on the gene transcription repressing effect by CRP (or CRP derivative) and the target gene expression inhibiting efficiency of the dxCas9-CRP system.

Specifically, the recombinant pdxCas9-CRP (or CRP derivative)-gRNA(R1) plasmid constructed in Experimental Example 3 and the GFP reporter plasmid constructed in Experimental Example 2 were transformed together into the strain E. *coli* MG1655 by heat shock, and then the strain was cultured on solid LB medium containing 25 µg/ml of chloramphenicol and 100 µg/ml of ampicillin, thereby selecting transformed strains. Then, the selected strains were cultured in liquid-LB medium containing 1 mM L-Rhamnose, and the cells cultured for 24 hours were imaged with a confocal fluorescence microscope. Thereafter, the GFP expression levels of individual cells were measured by fluorescence intensity (FIG. 12).

As a result, as can be confirmed in FIG. 12, the strain expressing CRP (or CRP derivative)-non-fused dxCas9 and gRNA(R1) together showed an approximate 75% or more reduction in GFP fluorescence value compared with the control strains expressing dxCas9 and non-targeting gRNA, indicating that the gene of dxCas9 itself had a transcriptional repression effect. Particularly, the dxCas9-CRP_{AR123} derivative-expressing strain showed an approximate 84% or more inhibition in GFP expression.

Therefore, the transcriptional repression effect of dxCas9 by the fusion of the CRP derivative could be validated, and the gene expression enhancement and inhibition experiments in Examples 1-1 and 1-2 verified that among the CRP derivatives, CRP_{AR123} had excellent transcriptional enhancement activity and showed the highest transcriptional repression activity, finally indicating that dxCas9-CRP_{AR123} was a novel gene expression control system.

### Example 2. Validation of simultaneous expression enhancement and inhibition control on multiple genes by dxCas9-CRP_{AR123}

An expression system for investigating the simultaneous expression enhancement and inhibition control effect on multiple genes was created by using the dxCas9-CRP_{AR123} system selected in Examples 1-1 and 1-2.

Specifically, the pMW7-P_{J23117}-GFP-P_{J23119}-mCherry plasmid and the pdxCas9-CRP_{AR123}-gRNA(A/R2) plasmid constructed in Experimental Examples 2 and 3 were co-expressed in *E*. *coli* MG1655 and the transformed cells were selected by the same **method** as in Example 1 above. Then, the selected *E*. *coli* cells were cultured for 24 hours in liquid-LB medium containing 1 mM L-Rhamnose, 25 µg/ml chloramphenicol, and 100 µg/ml ampicillin antibiotic. Thereafter, the cultured cells were harvested, and the cells were suspended in 1X PBS (pH 8.0) and dispensed at 200 µL per well into a 96-well plate. Next, the fluorescence intensity was measured by a microplate using light in a wavelength range that can detect the fluorescence of GFP (488/509 nm) and mCherry (587/610 nm) (FIG. 13).

As a result of observing the fluorescence intensity of the strain co-expressing dxCas9-CRP_{AR123}, gRNA(A), and gRNA(R2), as can be confirmed in FIG. 13, the corresponding strain showed an approximate 12.6-fold or higher increase in GFP fluorescence expression compared with the control strain by a composite of dxCas9-CRP_{AR123} and gRNA (A) and an approximate 93% reduction in mCherry expression compared with the control strain by a composite of dxCas9-CRP_{AR123} and gRNA(R2).

These results confirmed that the dxCas9-CRP_{AR123} system can control the expression of a target gene complementarily binding to the gRNA sequence, and the use of the dxCas9-CRP_{AR123} system of the present invention enables the targeting of respective multiple genes in cells, thereby simultaneously controlling the expression enhancement and repression of multiple genes.

While the present invention has been described with reference to the particular illustrative embodiments, a person skilled in the art to which the present invention pertains can understand that the present invention may be embodied in other specific forms without departing from the technical spirit or essential characteristics thereof. Therefore, the embodiments described above should be construed as being exemplified and not limiting the present invention. The scope of the invention should be construed that the meaning and scope of the appended claims rather than the detailed description and all changes or variations derived from the equivalent concepts fall within the scope of the present invention.

## Claims

1. A plasmid for controlling the expression of a target gene, the plasmid comprising dxCas9 and a CRP derivative,
wherein the CRP derivative is CRPAR1, CRPAR3, CRPAR23, or CRPAR123.

2. The plasmid of claim 1, wherein the CRP derivative is CRPAR123.

3. The plasmid of claim 2, wherein the CRPAR123 lacks a DNA-binding motif at the C-terminus in wild-type CRPWT consisting of SEQ ID NO: 5, comprises AR1, AR2, and AR3 domains, and consists of the 1st amino acid to 180th amino acid.

4. The plasmid of claim 2, wherein the CRPAR123 derivative comprises SEQ ID NO: 6.

5. The plasmid of any one of claims 1 - 4, wherein the dxCas9 comprises SEQ ID NO: 1.

6. The plasmid of any one of claims 1 - 5, wherein the controlling of the expression is enhancing or inhibiting the expression of the target gene.

7. The plasmid of any one of claims 1 - 6, wherein the dxCas9 is bound to a linker comprising the amino acid sequence of SEQ ID NO: 2.

8. The plasmid of any one of claims 1 - 7, wherein the plasmid comprises at least one guide RNA (gRNA) comprising a target gene sequence.

9. The plasmid of claim 8, wherein the guide RNA is at least one selected from the group consisting of the nucleotide sequences of SEQ ID NOS: 23 to 25.

10. A recombinant strain transformed with the plasmid of any one of claims 1 to 9.

11. The recombinant strain of claim 10, wherein the strain controls gene expression.

12. The recombinant strain of claim 10 or 11, wherein the strain enhances or inhibits the expression of a single gene or multiple target genes.

13. The recombinant strain of any one of claims 10 - 12, wherein the strain belongs to the genus Escherichia.

14. A method for preparing a recombinant strain controlling the expression of a target gene, the method comprising:
i) constructing a dxCas9-CRP system in which a dxCas9 protein is bound to a CRP protein;
ii) cloning a fluorescent reporter plasmid in the constructed dxCas9-CRP system;
iii) additionally cloning a guide RNA in the dxCas9-CRP system constructed in step ii); and
iv) transforming the dxCas9-CRP-gRNA into a strain.

15. A method for controlling the expression of a target gene, the method comprising applying a CRP derivative to dxCas9.
